# EUROPEAN PATENT APPLICATION

(11) **EP 3 395 309 A1**
(43) Date of publication of application: **31.10.2018**
(21) Application number: 16878025.2
(22) Date of filing: 25.07.2016
(51) Int. Cl.: A61F 13/15

(54) **MANUFACTURING METHOD AND MANUFACTURING DEVICE FOR CONTINUOUS SHEET COMPOSITE BODY PERTAINING TO ABSORBENT ARTICLES**

(30) Priority: 24.12.2015 JP 2015251161
(71) Applicant: Unicharm Corporation, Shikokuchuo-shi, Ehime 799-0111 (JP)
(72) Inventor: ISHIKAWA, Yoshihide, Kanonji-shi Kagawa 769-1602 (JP)
(74) Representative: Staeger & Sperling Partnerschaftsgesellschaft mbB
(86) International application number: PCT/JP2016/071732
(87) International publication number: WO 2017/110122

(57) **Abstract**

A method for manufacturing a composite body (1a) of a continuous sheet associated with an absorbent article (1), by attaching single-cut sheets (10) to a continuous sheet (20a, 24a) transported in a continuous manner at predetermined attachment pitches in a direction of transport of the continuous sheet (20a, 24a). Single-cut sheets (10) are transported in a direction of rotation of a rotating body (41), using a plurality of holding pads (51) provided to the rotating body (41) in line in the direction of rotation, by rotating the rotating body (41), the single-cut sheets (10) respectively held by the plurality of holding pads (51). The single-cut sheet (10) is delivered from the holding pad (51) to the continuous sheet (20a, 24a) transported along the direction of rotation, when each holding pad (51) passes a delivery position (Qout) in the direction of rotation. The delivery position including two delivery positions (Qout1, Qout2) disposed in the direction of rotation. A pitch of the holding pads (51, 51) adjacent in the direction of rotation is changed by moving the holding pads (51) in a rotation radius direction of the rotating body (41), in an area between a first delivery position (Qout1) and a second delivery position (Qout2) downstream therefrom.

## Description

### [Technical Field]

The present invention relates to a method and an apparatus for manufacturing a composite body of a continuous sheet associated with an absorbent article by attaching single-cut sheets to a continuous sheet at predetermined attachment pitches in a direction of transport.

### [Background Art]

Conventionally, in a production line of absorbent articles such as disposable diapers and sanitary napkins, single-cut sheets such as absorbent main bodies are attached to a continuous sheet at predetermined attachment pitches in a direction of transport (see PTL 1).

### [Citation List]

### [Patent Literature]

[PTL 1] Japanese Patent Application Publication No. 2005-298193

### [Summary of Invention]

### [Technical Problem]

The attachment pitches vary with product sizes of the absorbent articles. For example, the attachment pitch is large in M size and small in S size. Thus, in some product lines, apparatuses for attaching single-cut sheets to a continuous sheet are respectively prepared for product sizes of the absorbent articles. In these product lines, every time the size is changed, the apparatus is replaced with another apparatus corresponding to the size and used.

However, when the apparatuses respectively dedicated to product sizes are used, the cost of equipment increases, and replacement of the apparatuses becomes large-scale work, so that the rate of operation of the product line is greatly lowered.

The present disclosure has been made in view of the conventional issues as described above. An object of the present disclosure is to facilitate a change in attachment pitch for attaching single-cut sheets to a continuous sheet.

### [Solution to Problem]

A primary aspect of the disclosure is a method for manufacturing a composite body of a continuous sheet associated with an absorbent article, by attaching single-cut sheets to a continuous sheet transported in a continuous manner at predetermined attachment pitches in a direction of transport of the continuous sheet, the method comprising: transporting single-cut sheets in a direction of rotation of a rotating body, using a plurality of holding pads provided to the rotating body in line in the direction of rotation, by rotating the rotating body about a rotation axis, the single-cut sheets respectively held by the plurality of holding pads; and delivering each of the single-cut sheets from each of the holding pads to the continuous sheet transported along the direction of rotation, when each holding pad passes a delivery position in the direction of rotation, the delivery position including at least two alternatively selectable delivery positions disposed in the direction of rotation,
a pitch change area defined as an area in which a pitch of the holding pads adjacent to each other in the direction of rotation being changed by moving the holding pads in a direction of rotation radius of the rotating body, the pitch change area defined in the direction of rotation between a first delivery position and a second delivery position downstream from the first delivery position.

Further, an apparatus for manufacturing a composite body of a continuous sheet associated with an absorbent article, by attaching single-cut sheets to a continuous sheet transported in a continuous manner at predetermined attachment pitches in a direction of transport of the continuous sheet, the apparatus comprising: a transport device configured to transport single-cut sheets in a direction of rotation of a rotating body, using a plurality of holding pads provided to the rotating body in line in the direction of rotation, by rotating the rotating body about a rotation axis, the single-cut sheets respectively held by the plurality of holding pads; and a delivery device configured to deliver each of the single-cut sheets from each of the holding pads to the continuous sheet transported along the direction of rotation, when each holding pad passes a delivery position in the direction of rotation, the delivery position including at least two alternatively selectable delivery positions disposed in the direction of rotation, a pitch change area defined as an area in which a pitch of the holding pads adjacent to each other in the direction of rotation being changed by moving the holding pads in a direction of rotation radius of the rotating body, the pitch change area defined in the direction of rotation between a first delivery position and a second delivery position downstream from the first delivery position.

### [Advantageous Effects of Invention]

The present disclosure facilitates a change in attachment pitch for attaching single-cut sheets to a continuous sheet.

### [Brief Description of Drawings]

[Fig. 1] Fig. 1A and Fig. 1B are schematic plan views of diapers 1 in S size and M size, respectively.
[Fig. 2] Fig. 2 is a cross-sectional view taken along line II-II of Fig. 1A.
[Fig. 3] Fig. 3 is a schematic perspective view of a diaper 1.
[Fig. 4] Fig. 4 is a schematic diagram of a process carried out in a manufacturing apparatus 31 according to an embodiment of the present disclosure.
[Fig. 5] Fig. 5 is a schematic side view of a manufacturing apparatus 31 according to an embodiment of the present disclosure.
[Fig. 6] Fig. 6A is a schematic plan view of a holding pad 51, Fig. 6B is an arrow view taken along line B-B of Fig. 6A, and Fig. 6C is an arrow view taken along line C-C respectively of Fig. 6A.
[Fig. 7] Fig. 7 is a schematic side view of a rotating drum 41 to describe, in detail, operation patterns of an operation of moving a holding pad 51 in a direction of rotation radius Dr41, an operation of turning a holding pad 51 by 90 degrees, and an operation of moving a receiving blade 63 in a direction of rotation radius Dr41.
[Fig. 8] Fig. 8A is a timing diagram of an operation of moving a holding pad 51 in a direction of rotation radius Dr41, Fig. 8B is a timing diagram of an operation of turning a holding pad 51 by 90 degrees, and Fig. 8C is a timing diagram of an operation of moving a receiving blade 63 in a direction of rotation radius Dr41.
[Fig. 9] Fig. 9 is a schematic enlarged view of a transport roller 72 disposed on a second delivery position Qout2.

### [Description of Embodiments]

At least following matter will become clear from the descriptions of the present specification with reference to the accompanying drawings.

A method for manufacturing a composite body of a continuous sheet associated with an absorbent article, by attaching single-cut sheets to a continuous sheet transported in a continuous manner at predetermined attachment pitches in a direction of transport of the continuous sheet,
the method comprising:
transporting single-cut sheets in a direction of rotation of a rotating body, using a plurality of holding pads provided to the rotating body in line in the direction of rotation, by rotating the rotating body about a rotation axis, the single-cut sheets respectively held by the plurality of holding pads; and
delivering each of the single-cut sheets from each of the holding pads to the continuous sheet transported along the direction of rotation, when each holding pad passes a delivery position in the direction of rotation,
the delivery position including at least two alternatively selectable delivery positions disposed in the direction of rotation,
a pitch change area defined as an area in which a pitch of the holding pads adjacent to each other in the direction of rotation being changed by moving the holding pads in a direction of rotation radius of the rotating body, the pitch change area defined in the direction of rotation between a first delivery position and a second delivery position downstream from the first delivery position.

According to such a method for manufacturing a composite body of a continuous sheet associated with an absorbent article, the above-described pitch change area is set between the first delivery position and the second delivery position. Then, in this pitch change area, the pitch of the holding pads adjacent to each other is changed by moving the holding pad in the above-described direction of rotation radius . Accordingly, it is possible to easily change the attachment pitch of the single-cut sheets on the continuous sheet, by selecting the first delivery position or the second delivery position.

Further, the above-described pitch change can be stably performed, since this pitch is changed by moving the holding pad in the above-described direction of rotation radius.

In such a method for manufacturing a composite body of a continuous sheet associated with an absorbent article, it is desirable that the holding pads are provided at intervals, in the direction of rotation of the rotating body, each corresponding to a predetermined angle, and the first attachment position is spaced apart from the second attachment position in the direction of rotation by an angle equal to or larger than the predetermined angle.

According to such a method for manufacturing a composite body of a continuous sheet associated with an absorbent article, the first attachment position is spaced apart from the second attachment position in the direction of rotation by the angle equal to or larger than the above-described predetermined angle. Thus, it is possible to gradually carry out the operation of moving the holding pad in the direction of rotation radius between these first attachment position and second attachment position. As a result, this moving operation can be slowly carried out.

In such a method for manufacturing a composite body of a continuous sheet associated with an absorbent article, it is desirable that a first stop area and a second stop area are defined in the direction of rotation so that the first stop area, in which the holding pad is stopped without being moved in the direction of rotation radius, includes the first delivery position, and so that the second stop area, in which the holding pad is stopped without being moved in the direction of rotation radius, includes the second delivery position.

According to such a method for manufacturing a composite body of a continuous sheet associated with an absorbent article, the holding pad is not moved in the direction of rotation radius in the first stop area including the first delivery position. Thus, the holding pad can stably deliver the single-cut sheet to the continuous sheet at this first delivery position. Similarly, the holding pad is not moved in the direction of rotation radius in the second stop area including the second delivery position. Thus, the holding pad can stably deliver the single-cut sheet to the continuous sheet at this second delivery position.

In such a method for manufacturing a composite body of a continuous sheet associated with an absorbent article, it is desirable that the method further comprises: receiving a continuous sheet member using the holding pad when the holding pad passes a receiving position in the direction of rotation; and producing the single-cut sheets on the holding pads by cutting, with a cutter device, the continuous sheet member received by the holding pad, the cutter device includes: a cutter roll disposed downstream, in the direction of rotation, from the receiving position and outside the holding pad in the direction of rotation radius; and receiving blades respectively disposed at positions in the rotating body each between holding pads adjacent to each other in the direction of rotation, so as to receive a cutter blade of the cutter roll, when each of the receiving blades passes a position of the cutter roll in the direction of rotation, the continuous sheet member is cut with each receiving blade and the cutter blade of the cutter roll, to produce the single-cut sheets on the holding pads, the receiving blade is moved in the direction of rotation radius corresponding to its position in the direction of rotation, and the receiving blade does not protrude outside the holding pad in the direction of rotation radius at the receiving position, the first delivery position, and the second delivery position.

According to such a method for manufacturing a composite body of a continuous sheet associated with an absorbent article, the receiving blade does not protrude outside the holding pad in the direction of rotation radius at the receiving position, the first delivery position, and the second delivery position. It is possible to effectively prevent the receiving blade from damaging the above-described continuous sheet member and the above-described continuous sheet.

In such a method for manufacturing a composite body of a continuous sheet associated with an absorbent article, it is desirable that the holding pad has a holding surface directed outward in the direction of rotation radius to hold the single-cut sheet, the holding pad being provided so as to be turnable about an axis along the direction of rotation radius, during a time period in which the holding pad is moved in the direction of rotation from a position of the cutter roll to the first delivery position, the holding pad performs its turning operation about the axis, to change an orientation of the single-cut sheet, and the changed orientation is maintained until the holding pad passes the first delivery position and the second delivery position, and before the holding pad performs the turning operation, the holding pad starts to move outward in the direction of rotation radius, and at least the receiving blade adjacent on a side downstream, in the direction of rotation, from the holding pad starts to be moved inward in the direction of rotation radius.

According to such a method for manufacturing a composite body of a continuous sheet associated with an absorbent article, the holding pad carries out the above-described turning operation. Accordingly, when the single-cut sheet has a longitudinal direction and a width direction, it is possible to deliver the single-cut sheet, with its longitudinal direction replaced with its width direction between the above-described position of the cutter roll and the delivery position in the direction of rotation. Here, such a process is often necessary when an absorbent main body, which is a component of the absorbent article such as a disposable diaper and a sanitary napkin, is mounted onto another component. Accordingly, this process is appropriately used in manufacturing the absorbent article.

Further, the holding pad starts being moved outward in the direction of rotation radius before this holding pad carries out the above-described turning operation, and the receiving blade at least adjacent on the downstream side in the direction of rotation from this holding pad starts being moved inward in the direction of rotation radius. This can effectively prevent a possible malfunction at the time of the above-described turning operation, that is, the holding pad performing this turning operation can be prevented from interfering with the holding pads adjacent to each other in the direction of rotation and the receiving blade adjacent on the downstream side in the direction of rotation.

In such a method for manufacturing a composite body of a continuous sheet associated with an absorbent article, it is desirable that, by a time when the holding pad having passed the second delivery position reaches the receiving position, the holding pad performs another turning operation about the axis, and returns to its original orientation, and after the holding pad completes the other turning operation, the holding pad completes an operation of being moved inward in the direction of rotation radius to return to a position, in the direction of rotation radius, at which the holding pad should be positioned at the receiving position, and at least the receiving blade adjacent on a side upstream, in the direction of rotation, from the holding pad completes an operation of being moved outward in the direction of rotation radius to return to a position, in the direction of rotation radius, at which the receiving blade should be positioned at the receiving position.

According to such a method for manufacturing a composite body of a continuous sheet associated with an absorbent article, after the holding pad completes the other turning operation, the holding pad completes the operation of being moved inward to return to the position, in the direction of rotation radius, at which the holding pad should be positioned at the receiving position, and the above-described receiving blade completes the operation of moving outward to return to the position, in the direction of rotation radius, at which the above-described receiving blade should be positioned at the receiving position. This can effectively prevent a possible malfunction at the time of the above-described other turning operation, that is, the holding pad that carries out the above-described other turning operation can be prevented from interfering with the holding pads adjacent to each other in the direction of rotation and the receiving blade adjacent on the upstream side in the direction of rotation.

In such a method for manufacturing a composite body of a continuous sheet associated with an absorbent article, it is desirable that the receiving blade adjacent on a side upstream, in the direction of rotation, from the holding pad that is being moved in the pitch change area is positioned, in the direction of rotation radius, inside the position at which the receiving blade should be positioned at the receiving position, and the receiving blade is moved outward in the direction of rotation radius, in conjunction with an operation of the holding pad of being moved outward in the direction of rotation radius while being moved in the pitch change area.

According to such a method for manufacturing a composite body of a continuous sheet associated with an absorbent article, the receiving blade adjacent on the side upstream, in the direction of rotation, from the holding pad that is being moved in the pitch change area is moved outward in the direction of rotation radius, in conjunction with the operation of the holding pad of being moved outward in the direction of rotation radius. Accordingly, by carrying out, here, a part of the movement to the position outward in the direction of rotation radius at which the above-described receiving blade should be positioned at the receiving position, it is possible to slowly carry out this movement as a whole in a wider range in the direction of rotation. This can effectively prevent a possible trouble in the case of a rapid movement, for example, an increase in load of a driving device used to move the receiving blade in the direction of rotation radius.

Further, an apparatus for manufacturing a composite body of a continuous sheet associated with an absorbent article, by attaching single-cut sheets to a continuous sheet transported in a continuous manner at predetermined attachment pitches in a direction of transport of the continuous sheet, the apparatus comprising: a transport device configured to transport single-cut sheets in a direction of rotation of a rotating body, using a plurality of holding pads provided to the rotating body in line in the direction of rotation, by rotating the rotating body about a rotation axis, the single-cut sheets respectively held by the plurality of holding pads; and a delivery device configured to deliver each of the single-cut sheets from each of the holding pads to the continuous sheet transported along the direction of rotation, when each holding pad passes a delivery position in the direction of rotation, the delivery position including at least two alternatively selectable delivery positions disposed in the direction of rotation, a pitch change area defined as an area in which a pitch of the holding pads adjacent to each other in the direction of rotation being changed by moving the holding pads in a direction of rotation radius of the rotating body, the pitch change area defined in the direction of rotation between a first delivery position and a second delivery position downstream from the first delivery position.

Such an apparatus for manufacturing a composite body of a continuous sheet associated with an absorbent article can provide operational effects similar to those in the case of the above-described manufacturing method.

### === Present Embodiment ===

A method and an apparatus 31 for manufacturing a composite body 1a of a continuous sheet associated with an absorbent article 1 in an embodiment of the present disclosure are applied to, for example, a product line for disposable diapers 1.

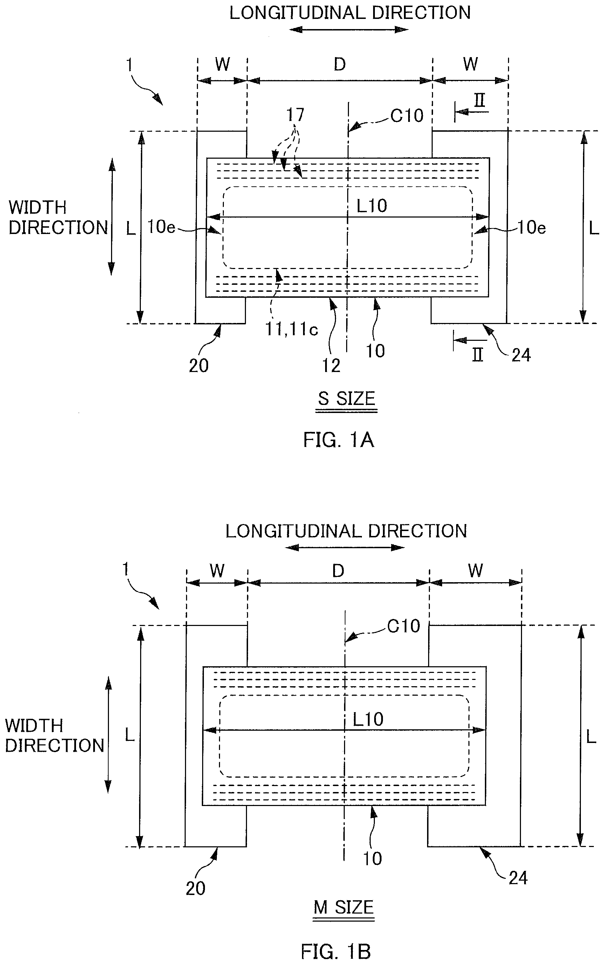

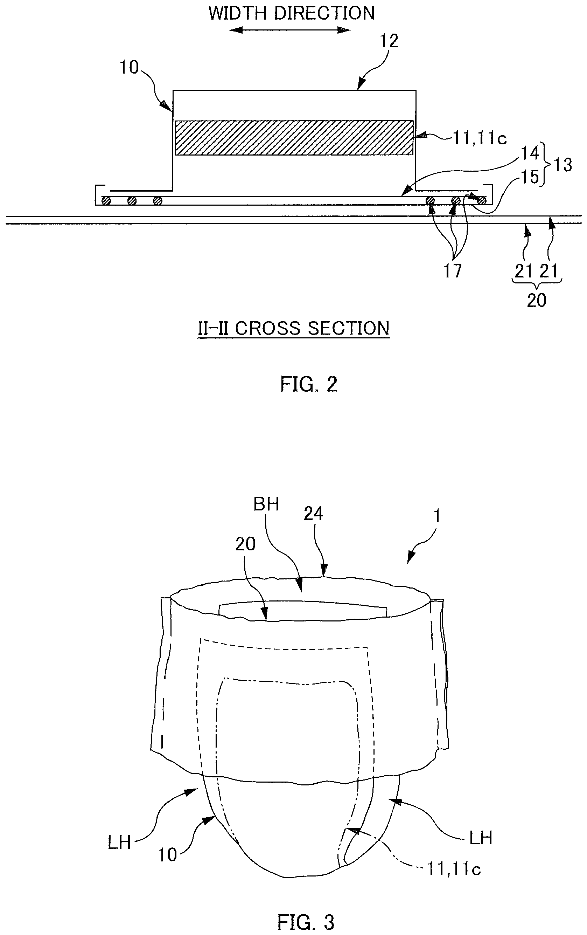

Fig. 1A to Fig. 3 are explanatory views of such a disposable diaper 1. Fig. 1A and Fig. 1B are schematic plan views of the diapers 1 in S size and M size, respectively. Fig. 2 is a cross-sectional view taken along line II-II in Fig. 1A. Fig. 3 is a schematic perspective view of the diaper 1.

The diaper 1 includes a front band member 20 that covers the front portion of a wearer, a back band member 24 that covers the back portion of the wearer, and an absorbent main body 10 that is placed on the crotch of the wearer and absorbs exudates such as urine. In its unfolded state in Fig. 1A, the front band member 20 and the back band member 24 are aligned in parallel with a distance D set therebetween, and the absorbent main body 10 is laid therebetween and both ends 10e, 10e in the longitudinal direction of the absorbent main body 10 are fixed to the members. Its appearance is in a substantially H-shape in plan view. Then, from this state, the diaper 1 is folded into two at a substantially center portion C10 in the longitudinal direction of the absorbent main body 10 serving as a folding position, and the band members 20, 24 opposed to one another are fastened at sites that should contact the flanks of the wearer in this two-folded state. Then, these band members 20, 24 are coupled to one another into an annular shape. This results in the diaper 1 when worn in which a waist opening BH and a pair of leg openings LH, LH are formed as illustrated in Fig. 3.

The diaper 1 formed into a pull-on diaper when a non-removable bonding structure such as welding is used, or formed into an open-type diaper when a removable bonding structure such as a fastening tape member (not illustrated) is used, as the above-described fastening structure. The components of the diaper 1 will be described below with reference to Fig. 1A and Fig. 2.

The absorbent main body 10 includes an absorbent body 11, a front-surface sheet member 12 that covers the absorbent body 11 from a skin side of the wearer, and a back-surface sheet member 13 that covers the absorbent body 11 from the opposite side of the front-surface sheet member 12 to double as an exterior of the diaper 1. The absorbent body 11 includes an absorbent core 11c, as a main body, which is obtained by forming liquid absorbent fibers such as pulp fibers into a substantially rectangular shape in plan view. This core 11c may contain a high absorbent polymer, and may be covered with a liquid permeable sheet such as a tissue. The front-surface sheet member 12 is, for example, a liquid permeable nonwoven fabric having a planar size larger than that of the absorbent body 11. The back-surface sheet member 13 is a liquid impermeable sheet having a planar size larger than that of the absorbent body 11, and one example thereof is the sheet 13 having a two-layer structure where a liquid impermeable leak-proof sheet 14 such as a polyethylene and an exterior sheet 15 such as a nonwoven fabric are attached one another. Then, in a state where the absorbent body 11 is sandwiched between these back-surface sheet member 13 and front-surface sheet member 12, the back-surface sheet member 13 and the front-surface sheet member 12 are attached one another into a frame shape at a portion extending outside from four sides of the absorbent body 11. This forms the absorbent main body 10.

As illustrated in Fig. 2, elastic members 17 such as elastic strings along the longitudinal direction may be interposed between the leak-proof sheet 14 and the exterior sheet 15 and fixed in a stretched state on both ends in a width direction of the back-surface sheet member 13. Then, these elastic members 17 form leg gather portions on the respective leg openings LH, LH of the diaper 1, to provide stretchability.

The front band member 20 and the back band member 24 are both formed of a soft sheet such as nonwoven fabric. Here, as illustrated in Fig. 2, two nonwoven fabrics 21, 21 are laminated to form the respective band members 20, 24. The respective band members 20, 24 are respectively attached and fixed to the corresponding ends 10e, 10e of the absorbent main body 10 in the longitudinal direction. Elastic members such as elastic strings may be fixed to each of the band members 20, 24 in a stretched state, and provide stretchability to these band members 20, 24.

Change in product size of such a diaper 1, that is, change in size, is made by mainly changing only dimensions associated with the band members 20, 24. For example, as compared with an S-size diaper illustrated in Fig. 1A, an M-size diaper illustrated in Fig. 1B has longer lengths L of the band members 20, 24, larger widths W of the band members 20, 24, and the longer distance D between the band members 20, 24. However, the absorbent main bodies 10 having the same specifications for dimensions (i.e., the length L10 and the width of the absorbent main body 10) are used, irrespective of the product size such as S size and M size.

The aforementioned diaper 1 is produced such that processing and bonding of components are carried out onto a base material which is a given component continuously flowing through the product line, to produce an intermediate product, and then, further processing and bonding of components are carried out onto this intermediate product, thereby completing the diaper 1. The manufacturing method and the manufacturing apparatus 31 according to an embodiment of the present disclosure serve one of such manufacturing processes. In the following, for convenience of explanation, a description will be given assuming that the front band member 20 and the back band member 24 have the same width W. However, in practice, as illustrated in Fig. 1A and Fig. 1B, there are many cases where the back band member 24 has a larger width.

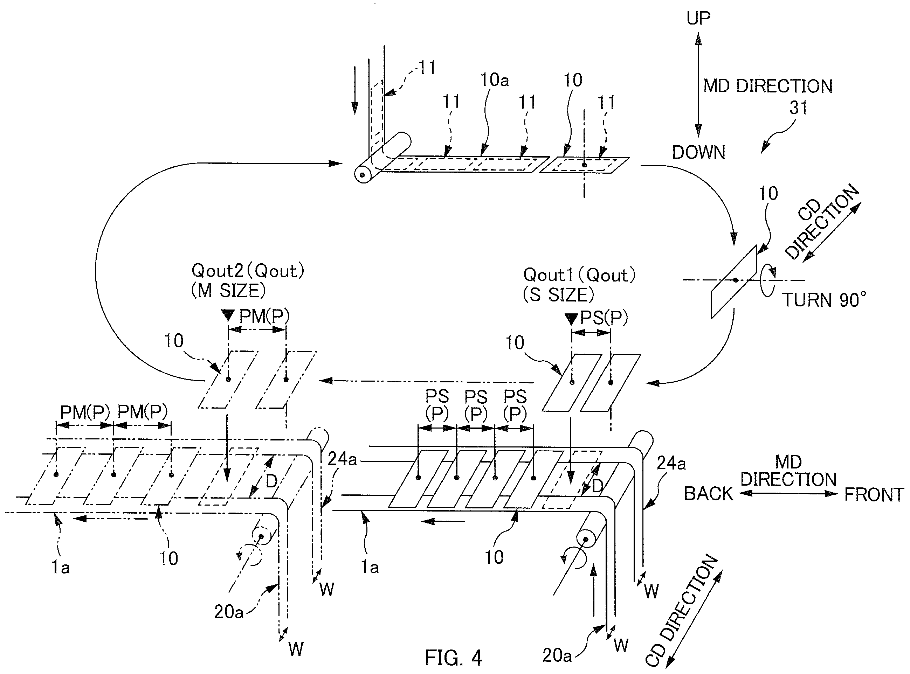

Fig. 4 is a schematic diagram of a process carried out in this manufacturing apparatus 31. In the following, a width direction of the manufacturing apparatus 31 is referred to as a "CD direction", and a direction perpendicular to this CD direction is referred to as an "MD direction". That is, the MD direction is a given direction in a plane perpendicular to the CD direction. There may be cases where two directions, orthogonal to each other, to the MD direction are referred to as an "up-down direction" and a "front-back direction".

In this manufacturing process, performed is a process of laying the absorbent main body 10 between band members 20, 24 in a pair and bonding them, thereby forming the intermediate product of the diaper 1 into a substantially H shape as in Fig. 1A.

In specific, as illustrated in Fig. 4, the pair of band members 20, 24 when being supplied to this manufacturing apparatus 31 is continuously transported in the form of the pair of continuous bodies 20a, 24a along the MD direction with the distance D provided therebetween in the CD direction. The absorbent main body 10 is also continuously transported in a form of a continuous body 10a continuous in the MD direction. That is, the front-surface sheet member 12 and the back-surface sheet member 13 constituting the absorbent main body 10 are in a state of the continuous sheet of the absorbent main body 10 continuous in the longitudinal direction. Then, while the absorbent body 11 is interposed between these front-surface sheet member 12 and back-surface sheet member 13, the absorbent bodies 11 are lined at intervals in the MD direction.

Accordingly, in this manufacturing apparatus 31, first, the absorbent-main-body continuous body 10a is divided at a position between the absorbent bodies 11, 11 along the CD direction, thereby producing the absorbent main bodies 10 whose longitudinal direction is directed in the MD direction. Then, while such an absorbent main body 10 is moved to a predetermined delivery position Qout, this absorbent main body 10 is turned 90 degrees about the center of its plane so that the longitudinal direction thereof is changed from the MD direction to the CD direction which is a direction in which the band-member continuous bodies 20a, 24a are aligned. Then, when the absorbent main body 10 reaches the delivery position Qout, the absorbent main body 10 is laid between the pair of band-member continuous bodies 20a, 24a which are along the CD direction and flow through this position Qout in the MD direction, at predetermined attachment pitches P, and both ends 10e, 10e thereof in the longitudinal direction are respectively attached to the band-member continuous bodies, so that the substantially ladder-type intermediate product 1a in Fig. 4 is produced, which is in a previous stage of the above-described substantially H shaped product in Fig. 1A. Then, a range of the process up to here is the range of the process handled by this manufacturing apparatus 31. Incidentally, in this example, the absorbent-main-body continuous body 10a corresponds to a "continuous sheet member", the absorbent main body 10 corresponds to a "single-cut sheet", the pair of band-member continuous bodies 20a, 24a corresponds to "continuous sheets", and the substantially ladder-like intermediate product 1a corresponds to a "composite body of a continuous sheet".

Here, when considering the process in this manufacturing process focusing on the product size, it is understood, from the comparison between Fig. 4 and Fig. 1A, that the length L of the band member 20, 24, which is the first condition among the aforementioned three conditions for determining the product size, is determined by the above-described attachment pitch P of the absorbent main body 10. It is also understood that the distance D between the band members 20, 24 and the width W of the band member 20, 24 of the diaper 1, which are other two conditions for determining the product size, are determined by the distance D between the pair of band-member continuous bodies 20a, 24a to be transported and the width W of the continuous body 20a, 24a.

Accordingly, as illustrated in Fig. 4, this manufacturing apparatus 31 is configured, to be able to manufacture two product sizes, i.e., S size and M size, such that two delivery positions Qout1, Qout2 for delivering the absorbent main bodies 10 to the band-member continuous bodies 20a, 24a are prepared and the attachment pitch P of the absorbent main bodies 10 can be changed between these delivery positions Qout1, Qout2. This manufacturing apparatus 31 is also configured such that the band-member continuous bodies 20a, 24a in pairs having dimensions W, D corresponding to the product sizes are able to run through the delivery positions Qout1, Qout2, respectively. Then, products different in product size are produced separately by alternatively selecting the delivery position Qout1 for S size and the delivery position Qout2 for M size.

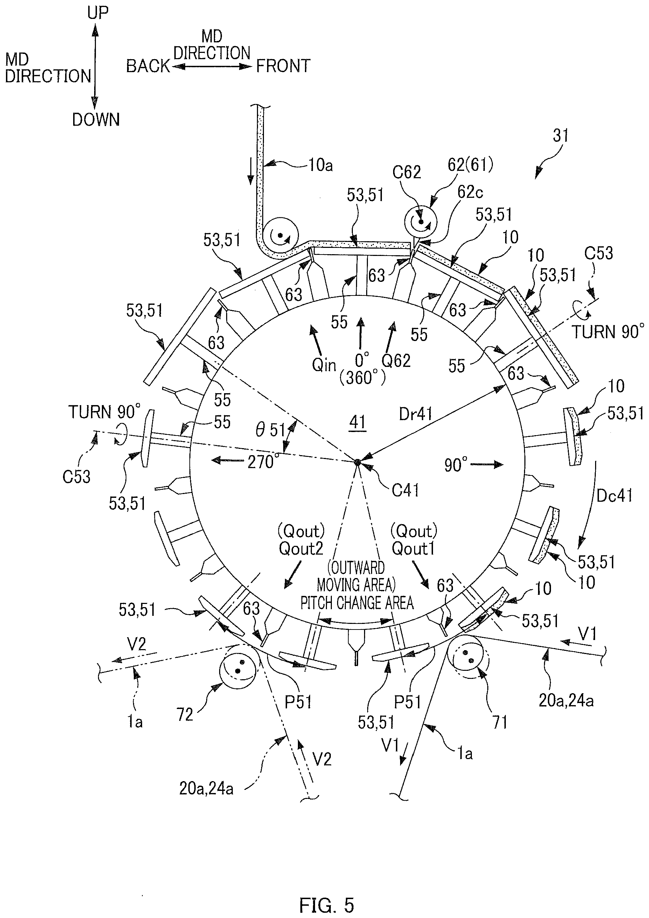

Fig. 5 is a schematic side view of the manufacturing apparatus 31 according to an embodiment of the present disclosure. In Fig. 5, the MD direction is a given direction along the paper surface of Fig. 5, and the CD direction is a direction passing through the paper surface of Fig. 5.

This manufacturing apparatus 31 includes a rotating drum 41 (corresponding to a rotating body and a transport device) . The rotating drum 41 rotates about a rotation axis C41 along the CD direction. A plurality of (for example, thirteen) holding pads 51, 51 ... are disposed, in line at intervals each corresponding to a predetermined angle θ51 (for example, 27 . 69 degree, and hereinafter also referred to as an arrangement angle θ51) in a direction of rotation Dc41 of the rotating drum 41, onto an outer peripheral portion of the rotating drum 41. Then, the respective holding pads 51, 51 ... each obtain a rotational force from a rotating operation of the rotating drum 41, thereby being rotated together with the rotating drum 41 in the direction of rotation Dc41.

Incidentally, as is apparent from Fig. 5, the direction of rotation Dc41 of the rotating drum 41 is included in the above-described MD direction. Accordingly, hereinafter, a description may also be given using "the direction of rotation Dc41" instead of the MD direction.

Such a holding pad 51 has a holding surface 53 in which a suction force is generated. This holding surface 53 is directed outward in the direction of rotation radius Dr41 of the rotating drum 41. The above-described absorbent-main-body continuous body 10a is transported toward a receiving position Qin which is set at a predetermined position in the direction of rotation Dc41. Accordingly, when the holding pad 51 passes through the receiving position Qin, the holding pad 51 receives the absorbent-main-body continuous body 10a with its holding surface 53 sucking one side of the absorbent-main-body continuous body 10a and holding it on the surface 53.

A cutter roll 62 of a cutter device 61 is disposed at a position Q62 positioned downstream from the receiving position Qin in the direction of rotation Dc41. The cutter roll 62 has a cutter blade 62c on its outer peripheral surface. Receiving blades 63 that receive the cutter blade 62c are disposed at respective positions between the holding pads 51, 51 in the direction of rotation Dc41 in the rotating drum 41. Then, every time such a receiving blade 63 passes the position Q62 of the cutter roll 62 in the direction of rotation Dc41, the cutter roll 62 is drivingly rotated so that the cutter blade 62c faces the receiving blade 63, thereby cutting a downstream end portion of the absorbent-main-body continuous body 10a to produce the absorbent main body 10 on the holding pad 51. Then the holding pad 51 is moved in the direction of rotation Dc41 while the absorbent main body 10 is being held onto the holding pad 51.

Meanwhile, the delivery positions Qout, Qout for delivering the absorbent main body 10 from the holding pad 51 to the band-member continuous bodies 20a, 24a are set alternatively selectable manner at two positions Qout, Qout located downstream from the position Q62 of the cutter roll 62 in the direction of rotation Dc41. Then, the holding pad 51 holding the absorbent main body 10, which is produced by cutting using the cutter roll 62, is moved to the selected delivery position Qout, while being turned 90 degrees so as to change the longitudinal direction of the absorbent main body 10 from the MD direction to the CD direction. Here, for example, when the upstream delivery position Qout1 (hereinafter, also referred to as a first delivery position Qout1) is selected, the pair of band-member continuous bodies 20a, 24a are placed beforehand on a transport roller 71 (corresponding to a delivery device) at this first delivery position Qout1 as indicated by a solid line in Fig. 5, so that the pair of band-member continuous bodies 20a, 24a is transported along the direction of rotation Dc41 at the first delivery position Qout1. Its transportation velocity V1 (m/second) in the direction of rotation Dc41 is controlled so as to be substantially equal to a velocity (m/second) of the holding pad 51 in the direction of rotation Dc41 at the first delivery position Qout1. Thus, when the holding pad 51 passes this first delivery position Qout1, the absorbent main body 10 is stuck on to be delivered to the pair of band-member continuous bodies 20a, 24a. Meanwhile, when a delivery position Qout2 (hereinafter also referred to as a second delivery position Qout2) located downstream from the above-described first delivery position Qout1 is selected, the pair of band-member continuous bodies 20a, 24a are placed beforehand on a transport roller 72 (corresponding to a delivery device) at the second delivery position Qout2, instead of the first delivery position Qout1, so that the pair of band-member continuous bodies 20a, 24a is transported along the direction of rotation Dc41 at this second delivery position Qout2 (see two-dot chain line in Fig. 5). Its transportation velocity V2 (m/second) in the direction of rotation Dc41 is controlled so as to be substantially equal to a velocity (m/second) of the holding pad 51 in the direction of rotation Dc41 at the second delivery position Qout2. Thus, when the holding pad 51 passes this second delivery position Qout2, the absorbent main body 10 is stuck on to be delivered to the above-described pair of band-member continuous bodies 20a, 24a.

Here, the attachment pitch P at which the absorbent main body 10 is attached to the band-member continuous bodies 20a, 24a is substantially equal to a pitch P51 of the holding pads 51, 51 adjacent to one another at the time of delivering. Thus, as long as such a configuration is made that the pitch P51 of the holding pads 51, 51 is changed between the first delivery position Qout1 and the second delivery position Qout2, it is possible to change the pitch P51 from an attachment pitch PS when the absorbent main body 10 is delivered at the first delivery position Qout1 to an attachment pitch PM when the absorbent main body 10 is delivered at the second delivery position Qout2.

Then, in an embodiment of the present disclosure, a pitch change area is set between the first delivery position Qout1 and the second delivery position Qout2 in the direction of rotation Dc41. In the pitch change area, the pitch P51 of the holding pads 51, 51 is changed by moving the holding pad 51 in the direction of rotation radius Dr41. In specific, an outward moving area, in which the holding pad 51 is moved outward in the direction of rotation radius Dr41, is set.

Accordingly, the holding pad 51 is moved outward in the direction of rotation radius Dr41 while passing the outward moving area. This increases the pitch P51 of the holding pads 51, 51 adjacent in the direction of rotation Dc41. That is, the pitch P51 after passing the outward moving area becomes larger than the pitch P51 before passing the outward moving area. As a result, the pitch P51 at the second delivery position Qout2 becomes larger than the pitch P51 at the first delivery position Qout1 positioned downstream from the first delivery position Qout1. This enables attachment of the absorbent main body 10 at the small attachment pitch PS for S size at the first delivery position Qout1 for S size, as well as attachment of the absorbent main body 10 at the large attachment pitch PM for M size at the second delivery position Qout2 for M size.

In this example, the distance D between the pair of band-member continuous bodies 20a, 24a that flow through the transport rollers 71, 72 at the respective delivery positions Qout1, Qout2 and the width W of the band-member continuous bodies 20a, 24a is set to dimensions corresponding to each of the product sizes at the delivery positions Qout1, Qout2. That is, the transport roller 71 is disposed at the first delivery position Qout1 for S size so as to flow the pair of band-member continuous bodies 20a, 24a having smaller width W for S size, and the distance D between the band-member continuous bodies 20a, 24a in a pair is set smaller. On the other hand, the transport roller 72 is also disposed at the delivery position Qout2 for M size so as to flow the pair of band-member continuous bodies 20a, 24a having larger width W for M size, and the distance D between the band-member continuous bodies 20a, 24a in a pair is set greater.

Accordingly, product size is changed by alternatively selecting the delivery positions Qout1, Qout2 through change in the above-described attachment pitches P and the dimensions W, D of the band-member continuous bodies 20a, 24a.

The manufacturing apparatus 31 has been briefly described above. In the following, those not having been described of the components 41, 61, 71, 72, and the like in the manufacturing apparatus 31 will be described.

### <<<Cutter Device 61>>>

With reference to Fig. 5, the cutter device 61 includes the cutter roll 62 having the cutter blade 62c, and the plurality of receiving blades 63, 63 ... supported on the rotating drum 41, as described above. Then, the cutter roll 62 is disposed outside the holding pad 51 in the direction of rotation radius Dr41, and the cutter roll 62 is driven to rotate about a rotation axis C62 along the CD direction. Each of the receiving blades 63 is rotated together with the rotating drum 41 in the direction of rotation Dc41 by the rotating operation of the rotating drum 41 in the direction of rotation Dc41.

Here, each of the receiving blades 63 is guided by an appropriate guiding member (not illustrated) such as a linear guide, so as to be able to reciprocate on a straight line along the direction of rotation radius Dr41. Such a receiving blade 63 is configured to be moved in the direction of rotation radius Dr41 corresponding to the position of the rotating drum 41 in the direction of rotation Dc41. This avoids various types of problems that may occur in association with the operation of turning the holding pad 51 by 90 degrees and the operation of moving the holding pad 51 in the direction of rotation radius Dr41.

That is, first, it is necessary that the receiving blade 63 is positioned substantially at the same position as that of the holding surface 53 of the holding pad 51 at the position Q62 of the cutter roll 62 in the direction of rotation Dc41, in order to cut the absorbent-main-body continuous body 10a. Whereas, after such cutting, if this state is maintained when the holding pad 51 is turned 90 degrees, the receiving blade 63 may interfere with the holding pad 51. Thus, the receiving blade 63 needs to be moved to escape inward in the direction of rotation radius Dr41 before such 90 degree turning, and here, the receiving blade 63 is operated accordingly.

Further, in this example, due to the aforementioned change in the pitch P51, the positions of the holding pad 51 in the direction of rotation radius Dr41 at the delivery positions Qout1, Qout2 are further inside in the direction of rotation radius Dr41, as compared with the position of the holding pad 51 in the direction of rotation radius Dr41 at the position Q62 of the cutter roll 62. Thus, if the position of the receiving blade 63 in the direction of rotation radius Dr41 at the position of the cutter roll 62 is maintained at the delivery position Qout1, Qout2 also, the receiving blade 63 may bump and damage the band-member continuous bodies 20a, 24a at the delivery position Qout1, Qout2. Consequently, before reaching the delivery position Qout1, Qout2, the receiving blade 63 needs to be moved to escape inward in the direction of rotation radius Dr41, and here, the receiving blade 63 is operated accordingly. Mainly based on these two reasons, the receiving blade 63 is configured to be movable in the direction of rotation radius Dr41 in this embodiment.

Such an operation of moving the receiving blade 63 in the direction of rotation radius Dr41 is implemented using, for example, a cam member (not illustrated). That is, for example, an annular groove (not illustrated) as a face cam is formed, so as to surround the rotation axis C41 of the rotating drum 41, on a vertical surface of the cam member immovably fixed to the ground side. A cam follower (not illustrated) engaged with this annular groove is provided to the receiving blade 63. Then, a cam curve, which forms a shape of the annular groove, is set to a shape corresponding to the pattern to carry out the moving operation in the direction of rotation radius Dr41 as described above. Accordingly, when the rotating drum 41 carries out the rotating operation, the receiving blade 63 is moved to a position in the direction of rotation radius Dr41 corresponding to the positions of the rotating drum 41 in the direction of rotation Dc41, based on the engagement of the annular groove serving as the face cam with the cam follower.

### <<<Rotating Drum 41>>>

With reference to Fig. 5, the rotating drum 41 rotates about the rotation axis C41 along the CD direction as described above. The plurality of holding pads 51, 51 ... are disposed and supported at intervals each corresponding to the aforementioned arrangement angle θ51 in the direction of rotation Dc41, onto the outer peripheral portion of the rotating drum 41. Then, the rotating drum 41 rotates using a servo motor or the like serving as a driving source. For example, in Fig. 5, the rotating drum 41 is driven to rotate clockwise in the direction of rotation Dc41, thereby also rotating the plurality of holding pads 51, 51 ... together with the rotating drum 41 in the above-described direction of rotation Dc41.

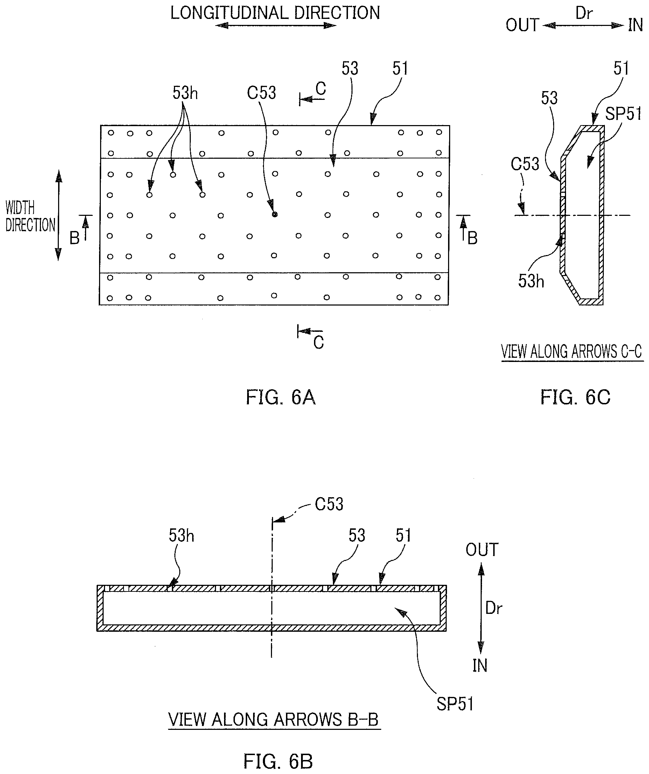

Fig. 6A is a schematic plan view of the holding pad 51, and Fig. 6B and Fig. 6C are an arrow view taken along line B-B and an arrow view taken along line C-C of Fig. 6A, respectively.

The holding pad 51 is a substantially rectangular plate-shaped member having the holding surface 53 where the absorbent main body 10 is held in a surface contact state, and as illustrated in Fig. 5, the holding surface 53 is directed outward in the direction of rotation radius Dr41 of the rotating drum 41. As illustrated in Fig. 6A, a plurality of intake holes 53h, 53h ... are formed substantially over the whole surface of the holding surface 53. These intake holes 53h, 53h ... are coupled to a negative-pressure source (not illustrated) such as a blower through a pressure chamber SP51 and an appropriate pipe passage (not illustrated) inside the holding pad 51. Accordingly, the suction force to hold the absorbent main body 10 on this holding surface 53 is caused by suction of the air from these intake holes 53h, 53h ...

The longitudinal direction of the holding surface 53 is aligned with the longitudinal direction of the holding pad 51. Thus, as illustrated in Fig. 5, by directing the longitudinal direction of each of the holding pads 51 in the direction of rotation Dc41 of the rotating drum 41 at the receiving position Qin and the position Q62 of the cutter roll 62, the absorbent main body 10 that is produced in a state where its longitudinal direction is directed in the direction of rotation Dc41 at the cutter roll 62 can be reliably held with the holding surface 53.

Further, as illustrated in Fig. 5 to Fig. 6C, the holding pad 51 is supported on the rotating drum 41 in such a manner as to be able to turn about a pivot axis C53 passing through the plane center of the holding surface 53 along the direction of rotation radius Dr41 of the rotating drum 41. Then, the holding pad 51 is turned about this pivot axis C53 by a turning angle of 90 degrees. This also turns the absorbent main body 10 on the holding pad 51 about the pivot axis C53 together with the holding pad 51. Thus, the longitudinal direction of the absorbent main body 10 is changed from the direction of rotation Dc41 to the CD direction. Then, as illustrated in Fig. 5, after the delivery of the absorbent main body 10 is completed at the delivery position Qout1 (or Qout2), the holding pad 51 performs a turning operation to be returned by 90 degrees, in order to receive the absorbent-main-body continuous body 10a again at the receiving position Qin. Accordingly, the longitudinal direction of the holding pad 51 is returned from the CD direction to the direction of rotation Dc41.

Meanwhile, this holding pad 51 is configured to be movable also in the direction of rotation radius Dr41. That is, each of the holding pads 51 is moved to a position in the direction of rotation radius Dr41, corresponding to a position in the direction of rotation Dc41. Accordingly, the holding pad 51 can change the pitch P51 of the holding pads 51 adjacent in the direction of rotation Dc41 as described above.

The holding pad 51 may interfere with the holding pad 51 adjacent in the direction of rotation Dc41, when being turned by 90 degrees as described above. However, this can also be prevented based on the aforementioned operation of moving in the direction of rotation radius Dr41. For example, as illustrated in Fig. 5, the longitudinal direction of the holding pad 51 is directed in the direction of rotation Dc41 at the position Q62 of the cutter roll 62 and the receiving position Qin in the direction of rotation Dc41. Thus, the holding pad 51 is adjacent to the holding pad 51 positioned upstream therefrom in the direction of rotation Dc41 with an extremely small clearance therebetween. Accordingly, when the holding pad 51 is turned 90 degrees about the pivot axis C53 in this state, corner portions at four corners of the holding pad 51 may interfere with the holding pad 51 adjacent thereto on the upstream side. However, in this respect, when the holding pad 51 is moved outward in the direction of rotation radius Dr41 before being turned 90 degrees as illustrated in Fig. 5, it is possible to avoid such interference between the holding pad 51 and the holding pad 51 adjacent thereto on the upstream side.

Such a 90 degree turning operation and a moving operation in the direction of rotation radius Dr41 of the holding pad 51 are implemented using, for example, a cam member and an arm member (both not illustrated) . For example, as illustrated in Fig. 5, a rod 55 along the direction of rotation radius Dr41 is fixed at the position of the pivot axis C53 on the surface, of the holding pad 51, facing the rotating drum 41. Then, the arm member (not illustrated) is provided to this rod 55 by spline fitting. That is, the rod 55 is allowed to perform a relative movement with respect to the arm member in the direction of rotation radius Dr41, but is restricted so as to be unable to perform relative rotation about the pivot axis C53. The arm member is supported onto the rotating drum 41 so as to be turnable about the above-described pivot axis C53 with an appropriate bearing member. Accordingly, when the arm member is turned about the pivot axis C53, the rod 55 is also turned about the pivot axis C53, thereby also turning the holding pad 51 about the pivot axis C53.

Meanwhile, the cam follower is provided to the arm member. This cam follower is engaged with a peripheral surface cam on an outer peripheral surface of the cam member (not illustrated) immovably fixed to the ground side. Then, an annular groove serving as the peripheral surface cam is formed so as to surround the rotation axis C41 of the rotating drum 41, and a cam curve, which is a shape of the annular groove, is set into a shape corresponding to the pattern of the 90 degree turning operation and the 90 degree return turning operation as described above. Accordingly, when the rotating drum 41 performs the rotating operation, the holding pad 51 performs the 90 degree turning operation and the 90 degree return turning operation based on the engagement between the annular groove, serving as the peripheral surface cam, and the cam follower.

A cam follower (not illustrated) is also provided onto the rod 55. Then, this cam follower is engaged with a face cam on a vertical surface of a cam member (not illustrated) immovably fixed to the ground side. Then, when the rotating drum 41 performs the rotating operation, the rod 55 performs the moving operation in the direction of rotation radius Dr41 based on this engagement. At such a time, the moving operation in the direction of rotation radius Dr41 is not transmitted to the above-described arm member, based on the above-described spline fitting. As a result, this arm member is not moved and only the rod 55 is moved. Then, with this movement of the rod 55, the holding pad 51 is moved in the direction of rotation radius Dr41.

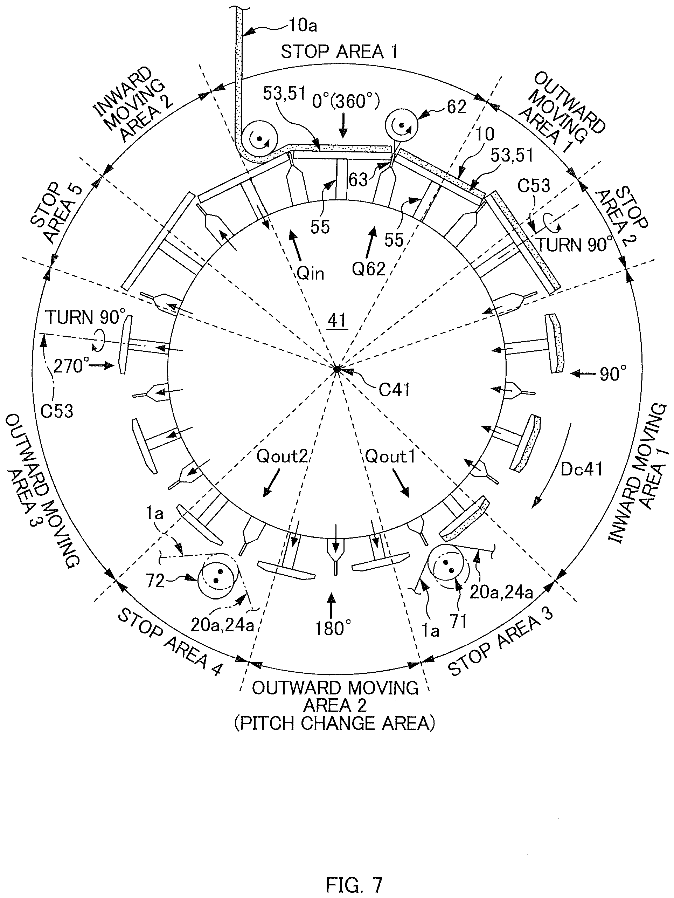

Fig. 7 is a schematic side view of the rotating drum 41 to describe in detail the patterns of the moving operation of the holding pad 51 in the direction of rotation radius Dr41, the 90 degree turning operation of the holding pad 51, and the moving operation of the receiving blade 63 in the direction of rotation radius Dr41.

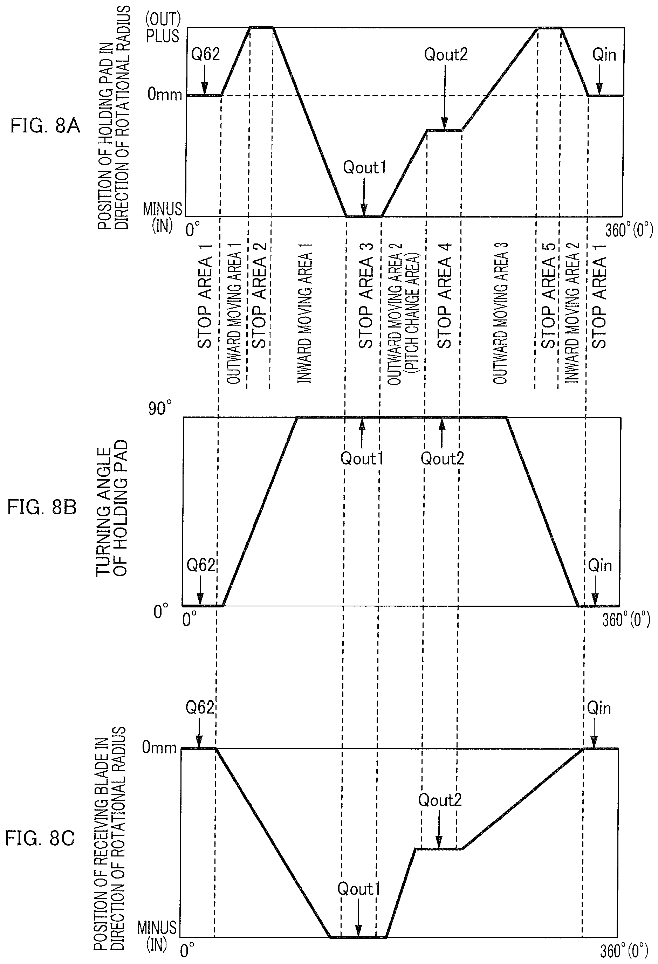

Fig. 8A is a timing diagram of the above-described moving operation of the holding pad 51 in the direction of rotation radius Dr41. Fig. 8B is a timing diagram of the 90 degree turning operation of the holding pad 51. Fig. 8C is a timing diagram of the moving operation of the receiving blade 63 in the direction of rotation radius Dr41. Horizontal axes of the timing diagrams in Fig. 8A and Fig. 8B are the position of the holding pad 51 in the direction of rotation Dc41. A horizontal axis of the timing diagram in Fig. 8C is the position of the receiving blade 63 in the direction of rotation Dc41. This position in the direction of rotation Dc41 is indicated by a rotation angle about the rotation axis C41 of the rotating drum 41. Then, the upper end position of the rotating drum 41 is 0 degree as illustrated in Fig. 5 and Fig. 7 and the rotating drum 41 is rotated in the clockwise direction. Accordingly, with respect to this rotation angle, angles from 0 degree to 360 degrees (0 degree) are allocated to positions when the rotating drum 41 is rotated one turn in the clockwise direction with the aforementioned upper end position serving as a starting point.

Meanwhile, vertical axes in Fig. 8A, Fig. 8B, and Fig. 8C are the position of the holding pad 51 in the direction of rotation radius Dr41, the turning angle about the pivot axis C53 of the holding pad 51, and the position of the receiving blade 63 in the direction of rotation radius Dr41, respectively. For the position of the holding pad 51 in the direction of rotation radius Dr41 in Fig. 8A, the position in the direction of rotation radius Dr41 at the receiving position Qin (Fig. 5 or Fig. 7) is defined as 0-mm position, serving as a reference position. Then, the position inside this reference position in the direction of rotation radius Dr41 is defined as a negative position, and the position outside the reference position in the direction of rotation radius Dr41 is defined as a positive position. Similarly, with respect to the position of the receiving blade 63 in the direction of rotation radius Dr41 in Fig. 8C as well, the position in the direction of rotation radius Dr41 at the receiving position Qin (Fig. 5 or Fig. 7) is defined as 0-mm position, serving as a reference position. Then, the position inside this reference position in the direction of rotation radius Dr41 is defined as a negative position, and the position outside the reference position in the direction of rotation radius Dr41 is defined as a positive position. With respect to the turning angle in Fig. 8B, a state where the longitudinal direction of the holding pad 51 is along the direction of rotation Dc41 is defined as 0 degree, and a state where the longitudinal direction is along the CD direction is defined as 90 degrees.

As illustrated in Fig. 7, in this example, the receiving position Qin is set at a position of about 340° (about 11 o'clock) in the direction of rotation Dc41. The cutter roll 62 is located at a position of about 15° (about one o'clock). The delivery position Qout1 for S size is set at a position of about 150° (about five o'clock). Further, the delivery position Qout2 for M size is set at a position of about 210° (about seven o'clock) located downstream from the delivery position Qout1.

Here, as illustrated in Fig. 7 and Fig. 8A, first, a stop area 1 is set so as to include the receiving position Qin in the direction of rotation Dc41. The "stop area" is an area in which the holding pad 51 is in a stopped state without being moved in the direction of rotation radius Dr41. Thus, when the holding pad 51 receives the absorbent-main-body continuous body 10a at the receiving position Qin, the holding pad 51 is not moved in the direction of rotation radius Dr41, so that the holding pad 51 can be stably received the absorbent-main-body continuous body 10a. Further, at this receiving position Qin, the receiving blade 63 is in such a state as not to protrude outside the holding pad 51 in the direction of rotation radius Dr41, as illustrated in Fig. 7. This reliably avoids the receiving blade 63 from hitting on to damage the absorbent-main-body continuous body 10a. From the view point of avoiding such damage more reliably, it is preferable that the receiving blade 63 is positioned inside the holding pad 51 in the direction of rotation radius Dr41 at the above-described receiving position Qin.

Further, the position Q62 of the cutter roll 62 is also included in this stop area 1. Thus, when the cutter roll 62 and the receiving blade 63 cut the absorbent-main-body continuous body 10a, the holding pad 51 holding the absorbent-main-body continuous body 10a is not moved in the direction of rotation radius Dr41, so that cutting can be stably carried out. As understood from Fig. 8C, the receiving blade 63 also is in this stop area 1 without being moved in the direction of rotation radius Dr41. Accordingly, this also effectively contributes to the stabilization of the above-described cutting at the position Q62.

Meanwhile, an outward moving area 1, a stop area 2, and an inward moving area 1 are set and aligned in this order toward the downstream side in the direction of rotation Dc41 in a range from this stop area 1 to the first delivery position Qout1. The "outward moving area" is an area in which the holding pad 51 is moved outward in the direction of rotation radius Dr41 and the "inward moving area" is an area in which the holding pad 51 is moved inward in the direction of rotation radius Dr41, as described above.

Then, the holding pad 51 that has passed the position Q62 of the cutter roll 62 in the direction of rotation Dc41 and holds the absorbent main body 10, is firstly turned 90 degrees about the pivot axis C53, so that the longitudinal direction of the absorbent main body 10 is changed from the direction of rotation Dc41 of the rotating drum 41 to the CD direction, while passing the outward moving area 1 and the stop area 2. Here, as illustrated in Fig. 8A and Fig. 8B, before this 90 degree turn, the holding pad 51 starts being moved outward in the direction of rotation radius Dr41. Thus, it is possible to avoid, beforehand, the aforementioned problem that the holding pad 51 may interfere with the holding pad 51 positioned upstream when being turned by 90 degrees.

Further, substantially at the same time when the holding pad 51 starts passing the outward moving area 1, at least this holding pad 51 and the receiving blade 63 adjacent thereto on the downstream side (preferably on the upstream side) in the direction of rotation Dc41 start being moved inward in the direction of rotation radius Dr41 (Fig. 8A and Fig. 8C). Accordingly, this receiving blade 63 starts being moved inward in the direction of rotation radius Dr41, as illustrated in Fig. 8C, before the 90 degree turn. As a result, it is also possible to avoid, beforehand, the problem that the holding pad 51 may interfere with the receiving blade 63 when being turned by 90 degrees.

Then, as illustrated in Fig. 7 and Fig. 8A, the holding pad 51 passes the inward moving area 1. During this passage, the holding pad 51 is moved inward in the direction of rotation radius Dr41 to the position corresponding to the attachment pitch PS for S size (Fig. 8A). This changes the pitch P51 of the holding pads 51, 51 adjacent in the direction of rotation Dc41 to the attachment pitch PS corresponding to the S size.

That is, as illustrated in Fig. 7, the pitch P51 of the holding pads 51, 51 is substantially the same as that of the length L10 (Fig. 1A) of the absorbent main body 10 in the longitudinal direction, at the receiving position Qin and the position Q62 of the cutter roll 62. Thus, the pitch P51 at these positions Qin, Q62 is different from the attachment pitch PS when the absorbent main body 10 is delivered at the first delivery position Qout1 for S size. Accordingly, it is necessary to change the pitch P51 of the holding pads 51, 51 to this attachment pitch PS, and such change is carried out in this inward moving area 1. More specifically, in this example, the pitch P51 of the holding pads 51, 51 at the receiving position Qin and the position Q62 of the cutter roll 62 is larger than the attachment pitch PS at the first delivery position Qout1, and thus the holding pad 51 is moved inward, to reduce the pitch P51 of the holding pads 51, 51 to the attachment pitch PS, in the inward moving area 1.

As illustrated in Fig. 7 and Fig. 8A, a stop area 3 (corresponding to a first stop area) is set, so as to include the first delivery position Qout1, at a position downstream from this inward moving area 1. Thus, when the holding pad 51 passes the first delivery position Qout1, this holding pad 51 is not moved in the direction of rotation radius Dr41. Accordingly, the holding pad 51 can stably deliver the absorbent main body 10 to the pair of band-member continuous bodies 20a, 24a at this first delivery position Qout1.

As illustrated in Fig. 8C, the above-described movement of the receiving blade 63 inward in the direction of rotation radius Dr41 is continued until almost immediately before entering the above-described stop area 3. This brings the receiving blade 63 in a state of not protruding outside the holding pad 51 in the direction of rotation radius Dr41 at the first delivery position Qout1, as illustrated in Fig. 7. Further, in this stop area 3, the receiving blade 63 is in a state where the movement thereof in the direction of rotation radius Dr41 is stopped in such a non-protruding state. Thus, it is reliably avoided that the receiving blade 63 hits and damages the band-member continuous bodies 20a, 24a at this first delivery position Qout1. From the view point of avoiding such damage more reliably, the receiving blade 63 is preferably positioned inside the holding pad 51 in the direction of rotation radius Dr41 at the above-described first delivery position Qout1.

Thereafter, as illustrated in Fig. 7 and Fig. 8A, the holding pad 51 is moved to the second delivery position Qout2. An outward moving area 2 is set as the aforementioned pitch change area between the stop area 3 and the second delivery position Qout2. Then, the holding pad 51 is moved outward in the direction of rotation radius Dr41 to a position corresponding to the attachment pitch PM for M size, while passing this outward moving area 2. As a result, the pitch P51 of the holding pads 51, 51 adjacent in the direction of rotation Dc41 is changed to the attachment pitch PM corresponding to the M size.

Here, the first delivery position Qout1 is preferably spaced apart from the second delivery position Qout2 by the aforementioned predetermined angle θ51 (Fig. 5) in the direction of rotation Dc41, that is, an angle equal to or greater than the arrangement angle θ51 of the holding pads 51 in the direction of rotation Dc41. In this example, the first delivery position Qout1 is spaced apart from the second delivery position Qout2 by a distance corresponding to about 2.3 times the angle of this arrangement angle θ51. Then, with such a configuration, the outward moving area 2 can be secured largely in the direction of rotation Dc41. Accordingly, the outward moving operation of the holding pad 51 that should be carried out in this outward moving area 2 can be carried out slowly. As a result, stabilization of this moving operation can be ensured.

Further, as illustrated in Fig. 7 and Fig. 8A, a stop area 4 (corresponding to a second stop area) is set so as to include the second delivery position Qout2 positioned on the side downstream from this outward moving area 2. Thus, the holding pad 51 is not moved in the direction of rotation radius Dr41 when this holding pad 51 passes the second delivery position Qout2. Accordingly, the holding pad 51 can stably deliver the absorbent main body 10 to the pair of band-member continuous bodies 20a, 24a at the second delivery position Qout2.

Furthermore, in this outward moving area 2, the receiving blade 63 is also moved outward in the direction of rotation radius Dr41 in conjunction with the outward movement of the holding pad 51. This allows the receiving blade 63 to be moved as slowly as possible when being moved outward to the reference position (i.e., 0-mm position), in the direction of rotation radius Dr41, at which the receiving blade 63 should be positioned at the receiving position Qin. That is, in Fig. 8C, the receiving blade 63 positioned in the stop area 3 should be moved outward to the 0-mm position in the direction of rotation radius Dr41 before finally reaching the receiving position Qin; however, if the receiving blade 63 is moved outward, even to a small extent, beforehand in conjunction with the outward movement of the holding pad 51 as described above, the receiving blade 63 is not required to be moved rapidly within a small angle range in the direction of rotation Dc41. As a result, it is possible to prevent a possible trouble in such a rapid movement, for example, an increase in load on the aforementioned face cam of the cam member used to drive the receiving blade 63. Accordingly, it is possible to remove factors responsible for deterioration of position accuracy of the receiving blade 63, such as wear of the face cam.

However, even when being moved outward in conjunction with the holding pad 51 as such, the receiving blade 63 stops its outward moving operation so that the receiving blade 63 does not protrude outside the holding pad 51 in the direction of rotation radius Dr41 at the second delivery position Qout2. Then, as illustrated in Fig. 7, in the aforementioned stop area 4, the receiving blade 63 is maintained in a state in which the movement thereof in the direction of rotation radius Dr41 is stopped in such a non-protruding state. Thus, it is reliably avoided that the receiving blade 63 hits and damages the band-member continuous bodies 20a, 24a at the second delivery position Qout2. From the view point of avoiding such damage more reliably, the receiving blade 63 is preferably positioned inside the holding pad 51 in the direction of rotation radius Dr41 at the above-described second delivery position Qout2.

Meanwhile, as illustrated in Fig. 7 and Fig. 8A, an outward moving area 3, a stop area 5, and an inward moving area 2 are provided in line in this order between the stop area 4 and the receiving position Qin. Then, the holding pad 51 carries out the return turning operation by 90 degrees, so that the longitudinal direction of this pad 51 is changed from the CD direction to the direction of rotation Dc41 of the rotating drum 41 by the time when reaching the receiving position Qin. At this time, this holding pad 51 is moved outward in the direction of rotation radius Dr41 so as not to interfere with the holding pad 51 adjacent on the downstream side in the direction of rotation Dc41. That is, the holding pad 51 is moved outward in the direction of rotation radius Dr41 while passing the outward moving area 3. Thus, the holding pad 51 is moved to a position in the direction of rotation radius Dr41 at which the holding pad 51 does not interfere with the holding pad 51 adjacent on the downstream side. More specifically, the holding pad 51 is moved to a position outside in the direction of rotation radius of the position of the holding pad 51 Dr41 at the receiving position Qin. During such a movement, the holding pad 51 carries out the return turning operation by 90 degrees . Then, after completing the 90 degree turn while passing through the outward moving area 3 and the stop area 5 adjacent on the side downstream from the outward moving area 3, the holding pad 51 completes the operation of moving inward in the direction of rotation radius Dr41 in the inward moving area 2. Accordingly, the holding pad 51 is moved to a position in the direction of rotation radius Dr41 at which the holding pad 51 should be positioned at the receiving position Qin. That is, the holding pad 51 is in a state of having returned to the 0-mm position, which is the aforementioned reference position. As illustrated in Fig. 8C, in this outward moving area 3, the stop area 5, and the inward moving area 2, at least the receiving blade 63 adjacent on the side upstream (preferably downstream), in the direction of rotation Dc41, from this holding pad 51 also carries out the operation of moving outward in the direction of rotation radius Dr, so that this receiving blade 63 returns to the reference position (i.e., 0-mm position) in the direction of rotation radius Dr41 at which the receiving blade 63 should be positioned at the receiving position Qin. This moving operation is also completed after the 90 degree turn is completed.

Accordingly, it is possible to effectively prevent a possible trouble at the time of the aforementioned 90 degree turning operation, that is, prevent the holding pad 51 that carries out the 90 degree turning operation from interfering with the holding pad 51 adjacent on the downstream side in the direction of rotation Dc41 and the receiving blades 63 adjacent in the direction of rotation Dc41.

Thereafter, the holding pad 51 enters the stop area 1 positioned downstream from the inward moving area 2. Here, this stop area 1 is the same as the stop area 1 provided so as to include the receiving position Qin which has been firstly described. Accordingly, the attachment of the next absorbent main body 10 is carried out by repeating the above-described process hereinafter.

### <<<Transport Rollers 71, 72>>>

As illustrated in Fig. 5, the transport rollers 71, 72 that transport the pairs of band-member continuous bodies 20a, 24a are respectively disposed on the delivery positions Qout1, Qout2 . Since both of them have substantially the same structure, a description will be given here of the transport roller 72 at the second delivery position Qout2 on the downstream side.

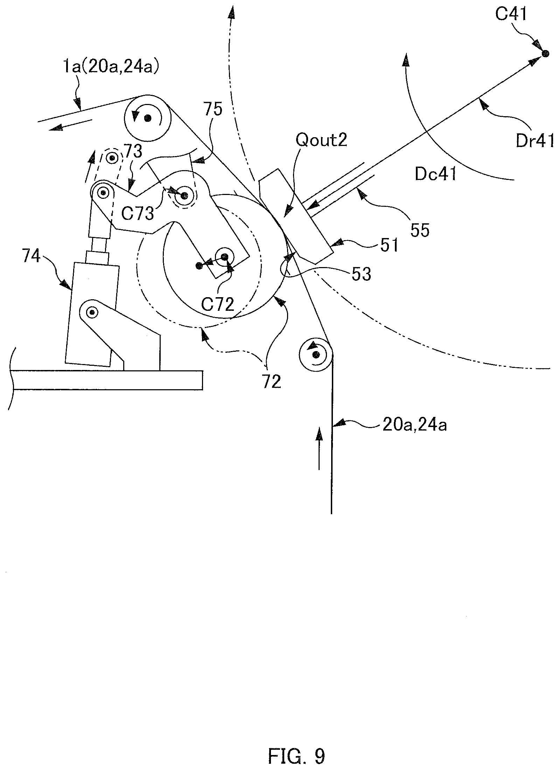

Fig. 9 is a schematic enlarged view of the transport roller 72.

The transport roller 72 is a driven roller 72 rotatably supported on one end portion of a predetermined arm member 73 and has a rotation axis C72 directed in the CD direction. The arm member 73 is supported on an appropriate stay member 75, with a position, serving as a supporting point C73, different from the rotation axis C72 of the transport roller 72. The arm member 73 is disposed swingably about this supporting point C73 in a plane parallel to the MD direction. Accordingly, the arm member 73 is swung using an appropriate actuator 74 such as an air cylinder so that the transport roller 72 is moved forward and backward between the second delivery position Qout2 in the direction of rotation Dc41 and a standby position (position of the transport roller 72 indicated by a two-dot chain line in Fig. 9) apart from the second delivery position Qout2. That is, out of the first and second delivery positions Qout1, Qout2, the transport roller 71 (or 72) at the delivery position Qout1 (or Qout2) that has not been selected can be moved backward to the standby position, while the transport roller 72 (or 71) at the selected delivery position Qout2 (or Qout1) can be moved forward to the delivery position Qout2 (or Qout1). As a result, the absorbent main body 10 can be reliably delivered to only the pair of band-member continuous bodies 20a, 24a that flow through the selected delivery position Qout2 (or Qout1).

This transport roller 72 is preferably made of such a soft material, e.g., sponge urethane rubber, that at least its outer peripheral portion is elastically deformable by contact pressure when being contacted by the holding pad 51. Then, with the use of such a material, for example, even when a rotation radius about the rotation axis C41 varies in the holding surface 53 with the position in the direction of rotation Dc41 at the time of the delivery of the absorbent main body 10 due to the shape of the holding surface 53 of the holding pad 51 as in this example in Fig. 9, a difference in this rotation radius can be promptly absorbed by the elastic deformation of the outer peripheral portion of the above-described transport roller 72. As a result, the absorbent main body 10 can be pressed to the band-member continuous bodies 20a, 24a over substantially the whole length in the direction of rotation Dc41.

### === Other Embodiments ===

Hereinabove, embodiments of the present disclosure have been described, however, the above embodiments of the present disclosure are simply to facilitate understanding of the present disclosure and are not in any way to be construed as limiting the present disclosure. The present disclosure may variously be changed or altered without departing from its gist and encompass equivalents thereof. For example, the following modifications are possible.

In an embodiment described above, the delivery positions Qout1, Qout2 are respectively set at two positions of the rotating drum 41 in the direction of rotation Dc41 in order to produce the absorbent articles in different sizes of S size and M size. However, it is not limited thereto. For example, it may be also possible that delivery positions Qout, Qout, and Qout are respectively set at three positions in the direction of rotation Dc41, each pitch change area is set each between the delivery positions Qout, Qout, and then, a given delivery position Qout is alternatively selected, to produce products in three different sizes or more such as an S size, an M size, and an L size.

In an embodiment described above, three of the length L of the band members 20, 24, the width W of the band members 20, 24, and the distance D between the pair of band members 20, 24 are changed in order to change the size. However, it is not limited thereto. For example, only one of the width W and the distance D may be changed to change the size. In some cases, neither of the width W and the distance D may be changed.

In an embodiment described above, the outward moving area 2 is described as one example of the pitch change area in which the pitch P51 of the holding pads 51, 51 is changed. However, it is not limited thereto. For example, the inward moving area may be used as the pitch change area.

In an embodiment described above, the disposable diaper 1 that absorbs exudates such as urine is given as an example of an absorbent article. However, it is not limited thereto. For example, the manufacturing method and the manufacturing apparatus 31 according to an embodiment of the present disclosure may be applied to manufacturing of a sanitary napkin that absorbs exudates such as menstrual blood.

In an embodiment described above, the absorbent main body 10 is given as an example of the single-cut sheet and the pair of band-member continuous bodies 20a, 24a is given as an example of the continuous sheet since the absorbent article is the three-piece type disposable diaper 1. However, it is not limited thereto. For example, the absorbent article may be a two-piece type disposable diaper. That is, this absorbent article may be a diaper that includes: an exterior sheet having a front section, a crotch section, and a back section, as a first component; and an absorbent main body 10 fixed to a skin side surface of this exterior sheet, as a second component. Further, this absorbent article may be a diaper having a form other than these.

An embodiment described above gives an example of such a configuration that the holding pad 51 is capable of being turned about the pivot axis C53. However, it is not limited thereto. The holding pad 51 may be incapable of being turned. That is, the absorbent main body 10 is not necessarily turned 90 degrees.

An embodiment described above gives an example of such a configuration that the receiving blade 63 of the cutter device 61 is movable in the direction of rotation radius Dr41 of the rotating drum 41. However, it is not limited thereto. The receiving blade 63 may be immovably fixed to the rotating drum 41.

In an embodiment described above, the receiving blade 63 is moved outward in conjunction with the outward movement of the holding pad 41 in the direction of rotation radius Dr41 in the pitch change area (outward moving area 2) in Fig. 7. However, it is not limited thereto. That is, the receiving blade 63 may be continued to be in a stopped state of not being moved outward in the direction of rotation radius Dr41 in this pitch change area from the stop area 3 adjacent on the upstream side of the pitch change area. Then, such a stopped state may be continued as it is to the stop area 4 (Fig. 7) adjacent on the downstream side in the direction of rotation Dc41. In this case, the receiving blade 63 is to be moved outward to the 0-mm position in the direction of rotation radius Dr41 at which this receiving blade 63 should be positioned at the receiving position Qin between the outward moving area 3 adjacent on the downstream side from this stop area 4 and the receiving position Qin.

In an embodiment described above, the operation of moving the holding pad 51 in the direction of rotation radius Dr41, the operation of turning the holding pad 51 by 90 degrees, and the operation of moving the receiving blade 63 in the direction of rotation radius Dr41 are respectively driven by the cam members. However, it is not limited thereto. For example, electric motors such as servo motors may be respectively provided to the holding pads 51, so as to perform the above-described moving operations and 90-degree turning operation, as well as electric motors such as servo motors may be respectively provided to the receiving blades 63 so as to perform the above-described moving operation .

### [Reference Signs List]

1 disposable diaper (absorbent article), 1a intermediate product (composite body of continuous sheet),
10 absorbent main body (single-cut sheet), 10e end,
10a absorbent-main-body continuous body (continuous sheet member),
11 absorbent body, 11c absorbent core,
12 front-surface sheet member, 13 back-surface sheet member,
14 leak-proof sheet, 15 exterior sheet,
17 elastic member,
20 front band member, 20a band-member continuous body (continuous sheet), 21 nonwoven fabric,
24 back band member, 24a band-member continuous body (continuous sheet),
31 manufacturing apparatus,
41 rotating drum (transport device),
51 holding pad, 53 holding surface, 53h intake hole, 55 rod
61 cutter device, 62 cutter roll, 62c cutter blade, 63 receiving blade,
71 transport roller, 72 transport roller (delivery device),
73 arm member, 74 actuator, 75 stay member,
BH waist opening, LH leg opening,
SP51 pressure chamber
C10 substantially center portion, C41 rotation axis,
C53 pivot axis, C62 rotation axis, C72 rotation axis, C73 supporting point,
Qin receiving position, Q62 position of cutter roll,
Qout delivery position,
Qout1 first delivery position, Qout2 second delivery position,

## Claims

1. A method for manufacturing a composite body of a continuous sheet associated with an absorbent article, by attaching single-cut sheets to a continuous sheet transported in a continuous manner at predetermined attachment pitches in a direction of transport of the continuous sheet,
the method comprising:
transporting single-cut sheets in a direction of rotation of a rotating body, using a plurality of holding pads provided to the rotating body in line in the direction of rotation, by rotating the rotating body about a rotation axis, the single-cut sheets respectively held by the plurality of holding pads; and
delivering each of the single-cut sheets from each of the holding pads to the continuous sheet transported along the direction of rotation, when each holding pad passes a delivery position in the direction of rotation,
the delivery position including at least two alternatively selectable delivery positions disposed in the direction of rotation,
a pitch change area defined as an area in which a pitch of the holding pads adjacent to each other in the direction of rotation being changed by moving the holding pads in a direction of rotation radius of the rotating body, the pitch change area defined in the direction of rotation between a first delivery position and a second delivery position downstream from the first delivery position.

2. A method for manufacturing a composite body of a continuous sheet associated with an absorbent article according to claim 1, wherein
the holding pads are provided at intervals, in the direction of rotation of the rotating body, each corresponding to a predetermined angle, and
the first attachment position is spaced apart from the second attachment position in the direction of rotation by an angle equal to or larger than the predetermined angle.

3. A method for manufacturing a composite body of a continuous sheet associated with an absorbent article according to claim 1 or 2, wherein
a first stop area and a second stop area are defined in the direction of rotation
so that the first stop area, in which the holding pad is stopped without being moved in the direction of rotation radius, includes the first delivery position, and
so that the second stop area, in which the holding pad is stopped without being moved in the direction of rotation radius, includes the second delivery position.

4. A method for manufacturing a composite body of a continuous sheet associated with an absorbent article according to any one of claims 1 to 3, wherein
the method further comprises:
receiving a continuous sheet member using the holding pad when the holding pad passes a receiving position in the direction of rotation; and
producing the single-cut sheets on the holding pads by cutting, with a cutter device, the continuous sheet member received by the holding pad,
the cutter device includes:
a cutter roll disposed downstream, in the direction of rotation, from the receiving position and outside the holding pad in the direction of rotation radius; and
receiving blades respectively disposed at positions in the rotating body each between holding pads adjacent to each other in the direction of rotation, so as to receive a cutter blade of the cutter roll,
when each of the receiving blades passes a position of the cutter roll in the direction of rotation, the continuous sheet member is cut with each receiving blade and the cutter blade of the cutter roll, to produce the single-cut sheets on the holding pads,
the receiving blade is moved in the direction of rotation radius corresponding to its position in the direction of rotation, and
the receiving blade does not protrude outside the holding pad in the direction of rotation radius at the receiving position, the first delivery position, and the second delivery position.

5. A method for manufacturing a composite body of a continuous sheet associated with an absorbent article according to claim 4, wherein
the holding pad has a holding surface directed outward in the direction of rotation radius to hold the single-cut sheet, the holding pad being provided so as to be turnable about an axis along the direction of rotation radius,
during a time period in which the holding pad is moved in the direction of rotation from a position of the cutter roll to the first delivery position,
the holding pad performs its turning operation about the axis, to change an orientation of the single-cut sheet, and
the changed orientation is maintained until the holding pad passes the first delivery position and the second delivery position, and
before the holding pad performs the turning operation,
the holding pad starts to move outward in the direction of rotation radius, and
at least the receiving blade adjacent on a side downstream, in the direction of rotation, from the holding pad starts to be moved inward in the direction of rotation radius.

6. A method for manufacturing a composite body of a continuous sheet associated with an absorbent article according to claim 5, wherein
by a time when the holding pad having passed the second delivery position reaches the receiving position,
the holding pad performs an other turning operation about the axis, and returns to its original orientation, and
after the holding pad completes the other turning operation,
the holding pad completes an operation of being moved inward in the direction of rotation radius to return to a position, in the direction of rotation radius, at which the holding pad should be positioned at the receiving position, and
at least the receiving blade adjacent on a side upstream, in the direction of rotation, from the holding pad completes an operation of being moved outward in the direction of rotation radius to return to a position, in the direction of rotation radius, at which the receiving blade should be positioned at the receiving position.

7. A method for manufacturing a composite body of a continuous sheet associated with an absorbent article according to any one of claims 4 to 6, wherein
the receiving blade adjacent on a side upstream, in the direction of rotation, from the holding pad that is being moved in the pitch change area is positioned, in the direction of rotation radius, inside the position at which the receiving blade should be positioned at the receiving position, and
the receiving blade is moved outward in the direction of rotation radius, in conjunction with an operation of the holding pad of being moved outward in the direction of rotation radius while being moved in the pitch change area.

8. An apparatus for manufacturing a composite body of a continuous sheet associated with an absorbent article, by attaching single-cut sheets to a continuous sheet transported in a continuous manner at predetermined attachment pitches in a direction of transport of the continuous sheet,
the apparatus comprising:
a transport device configured to transport single-cut sheets in a direction of rotation of a rotating body, using a plurality of holding pads provided to the rotating body in line in the direction of rotation, by rotating the rotating body about a rotation axis, the single-cut sheets respectively held by the plurality of holding pads; and
a delivery device configured to deliver each of the single-cut sheets from each of the holding pads to the continuous sheet transported along the direction of rotation, when each holding pad passes a delivery position in the direction of rotation,
the delivery position including at least two alternatively selectable delivery positions disposed in the direction of rotation,
a pitch change area defined as an area in which a pitch of the holding pads adjacent to each other in the direction of rotation being changed by moving the holding pads in a direction of rotation radius of the rotating body, the pitch change area defined in the direction of rotation between a first delivery position and a second delivery position downstream from the first delivery position.
